# EUROPEAN PATENT SPECIFICATION

(11) **EP 1 750 634 B1**
(45) Date of publication and mention of the grant of the patent: **03.11.2010**
(21) Application number: 05713238.3
(22) Date of filing: 08.02.2005
(51) Int. Cl.: A61F 13/15, B41J 3/54

(54) **TWO-STEP CONTACT AND NON-CONTACT PRINTING**
ZWEISTUFENKONTAKT UND KONTAKTLOSER DRUCK
IMPRESSION PAR CONTACT ET PAR NON CONTACT S'EFFECTUANT EN DEUX ETAPES

(30) Priority: 31.03.2004 US 813873
(43) Date of publication of application: 14.02.2007
(73) Proprietor: KIMBERLY-CLARK WORLDWIDE, INC., Neenah, WI 54956 (US)
(72) Inventor: COSTELLO, John, Patrick, Neenah, WI 54956 (US); SCHMID, John, Jerome, Sherwood, WI 54169 (US); JOHNSON, Eric, Donald, Larsen, WI 54947 (US)
(74) Representative: Mabey, Katherine Frances
(86) International application number: PCT/US2005/004151
(87) International publication number: WO 2005/102237

(56) References cited:
- EP-A- 0 624 477
- US-A- 3 913 719
- US-A- 4 423 676
- US-A1- 2003 088 228
- US-A1- 2004 058 130
- PATENT ABSTRACTS OF JAPAN vol. 1999, no. 10, 31 August 1999 (1999-08-31) -& JP 11 138915 A (NOKEG & G OPT ELECTRONICS KK; TOPPAN FORMS CO LTD), 25 May 1999 (1999-05-25)
- PATENT ABSTRACTS OF JAPAN vol. 017, no. 316 (M-1430), 16 June 1993 (1993-06-16) -& JP 05 031966 A (CANON INC), 9 February 1993 (1993-02-09)

## Description

### BACKGROUND OF THE INVENTION

Graphics are commonly printed on articles of manufacture to increase their anesthetic appeal_ One example of this is graphics or images printed on absorbent garments, such as infant and children's diapers and training pants. The outer covers of these articles are frequently imprinted with brightly colored images in the form of designs and characters that are pleasing to the child and caregiver. Images may also be printed on other absorbent articles such as feminine healthcare products, adult incontinence garments and bandages. However, graphics are not exclusive to personal care products, and could be, for example, applied to a cleaning wipe or sheet product.

Traditionally, printing the outer cover material or other portion of an article with an image has been carried out by flexographic printing, rotogravure printing, screen printing, offset printing, or other types of contact printing techniques prior to assembly of the absorbent article (i.e., the printing is done "off-line"). Typically, the image is printed on the outer cover material before being moved or shipped to the location where the absorbent article Is assembled. While printing an image during the manufacture of absorbent articles (i.e., printing "on-line"), by use of traditional contact printing processes, is possible, it has traditionally not been practical. Contact printing on-line can be difficult, time-consuming, and costly. These problems can be exacerbated when there is a desire to change the printed image. Changing the image typically requires shutting down the entire assembly process and installing new print rolls, screens, or plates. Additionally, contact printing on-line may create a significant amount of waste and delay during startup, shutdown, and other transitory process changes and therefore does not lend itself to the on-line manufacture of mass produced absorbent articles.

Non-contact printing systems are known to provide flexibility in image printing and typically include ink jet printing, wax jet printing, bubble jet printing, laser jet printing, and the like. Changing from one image to the next may be done rapidly without stopping the process. Drop on demand piezoelectric ink jet printing apparatus have been used to apply inks to a variety of substrates. Generally, a drop on demand piezoelectric ink jet printing apparatus discharges small individual droplets of ink onto a substrate in a predetermined pattern. In this type of apparatus, the print head does not contact the web on which it prints.

EP 0624477, US 4,423,676 and US 3,913,719 all disclose a method of printing a moving substrate comprising: supplying a moving substrate to a first converting operation; contact printing at least one first graphic on the moving substrate; and supplying the moving substrate with the first graphic to a second converting operation, and non-contact printing at least one second graphic on the moving substrate wherein both the first and second graphics are visible to the naked eye. US 2004/0058130 discloses color printing of graphics on diapers involving combinations of contact and non-contact printing methods.

However when the substrate is running under the print head at higher speed (e.g., 0.508 m/sec (100 feet per minute) or greater), it is very difficult for the print head to be controlled or supply an adequate amount of ink such that a large number of dots per inch (dpi)¹ can be accurately applied. This is particularly true where, as a result of the operation of the absorbent garment assembly apparatus, the image must be formed in a single pass of the material under the print heads. Non-contact printing is further complicated when wider patterns are desired. Typically when using a non-contact printer to create a wide pattern, additional banks of print heads are required to achieve the graphics desired. Consequently, while there has been progress in the area of non-contact printing, the printers have been limited in that they were not able to produce an image of a commercially acceptable quality at higher speeds encountered in the assembly of an absorbent garment such as a diaper or training pant.

Additionally, on-line non-contact printing can result in significant waste. Specifically, when a substrate, intended for use as a component of an absorbent article, encounters a printing failure, the entire absorbent article is usually discarded as defective. This increases the amount of waste that results from printing failures.

In view of the aforementioned problems, there arises the need for a method of printing high quality images on substrates that is cost effective and minimizes waste associated with printing failures.

### SUMMARY OF THE INVENTION

In response to the discussed difficulties and problems encountered previously, the present invention provides a method of printing a moving substrate, in accordance with claim 1.

In various embodiments, a gravure printer, flexographic printer, offset printer, or screen printer may be used for the contact printing. A wax Jet printer, ink jet printer, laser jet printer, or bubble jet printer may be used for the non-contact printing. The first graphic ¹ 1 inch = 2.54 cm and second graphic may jointly form a story line. The moving substrate may be traveling at least 0.508 metre/see (100 feet per minute) during the non-contact printing.

In various embodiments, at least one third graphic may be non-contact printed on the moving substrate and at least a portion of the third graphic may at least partially overprint the first graphic. The second graphic may at least partially overprint the first graphic. The first graphic and the second graphic may jointly form a master graphic.

The second converting operation produces disposable absorbent garments and the moving substrate forms an outer cover of the garments.

The substrate may be a laminate made of a film layer and a nonwoven layer and the first graphic may be printed on the film layer and the second graphic may be printed on the nonwoven layer. Alternatively, the substrate may be a laminate made of a film layer and a nonwoven layer and the first graphic may be printed on the nonwoven layer and the second graphic may be printed on the nonwoven layer. Alternatively, the substrate may be a laminate made of a film layer and a nonwoven layer and the first graphic may be printed on the film layer and the second graphic may be printed on the film layer.

One embodiment for printing an outer cover for an absorbent garment includes the steps of: supplying a moving first substrate to a first printing operation, the first moving substrate comprising a film; contact printing at least one first graphic on the first moving substrate in the first printing operation using a gravure roll printer or flexographic printer; laminating a second moving substrate to the first moving substrate to form an outer cover, the second moving substrate comprising a nonwoven web and the outer cover defining a width: supplying the outer cover to a second printing operation; non-contact printing at least one second graphic on the outer cover in the second printing operation using a wax jet printer, Ink jet printer, bubble jet printer, or laser jet printer, the first graphic spanning at least 60% of the width of the outer cover and being visible to the naked eye, the second graphic being positioned within the center third of the width of the outer cover and being visible to the naked eye; and joining the outer cover with an absorbent and a liner to produce an absorbent garment.

The absorbent garment may have a front waist region, a back waist region, and a crotch region connecting the front waist region and the back waist region. In such a garment, the second graphic may be positioned within the front waist region. Alternatively. the second graphic may be positioned within the back waist region. Alternatively, the outercover may have two or more second graphics arranged such that at least one second graphic is positioned within the front waist region and at least one second graphic is positioned within the back waist region of the absorbent garment.

One embodiment of printing an outer cover for an absorbent garment includes the steps of: laminating a first substrate made of film to a second substrate made of a nonwoven to form an outer cover, the outercover defining a film side, a nonwoven side opposite the film side, and a width; supplying the outer cover to a printing process and contact printing at least one first graphic on the nonwoven side; supplying the outer cover to a converting operation, the converting operation combining the outer cover with an absorbent assembly to form an absorbent garment: non-contact printing at least one second graphic on the nonwoven side in the converting operation, the first graphic spanning at least 60% of the width of the outer cover and being visible to the naked eye, the second graphic being positioned within the center third of the width of the outer cover, and both first and second graphics being visible to the naked eye.

### BRIEF DESCRIPTION OF THE DRAWINGS

**FIG. 1** representatively illustrates a plan view of one embodiment of an exemplary method.
**FIG. 2** representatively illustrates a vertical section view of an exemplary gravure printing process.
**Fig. 3** representatively illustrates a vertical section view of an exemplary wax jet printing process.
**Fig. 4** representatively illustrates a perspective view of an exemplary diaper.
**FIG. 5** representatively illustrates a plan view of one embodiment of a substrate including exemplary graphics.
**FIG. 6** representatively Illustrates a plan view of one embodiment of a substrate including exemplary graphics.
FIG. 7 representatively illustrates a plan view of one embodiment of a substrate including exemplary graphics.
**FIG. 8** representatively illustrates a plan view of one embodiment of a substrate including exemplary graphics.

### DETAILED DESCRIPTION OF THE DRAWINGS

The present invention relates to a method of printing substrates. More specifically, one aspect relates to a method of printing a first graphic on a moving substrate using a contact printing method, such as, for example, flexographic printing, rotogravure printing, offset printing, lithographic printing, screen printing, or the like. The method also involves printing the substrate using a non-contact printing method, such as, for example, wax jet printing, ink jet printing, bubble jet printing, laser jet printing, or the like. The method further includes the step of producing a disposable absorbent garment with one or more of the printed substrates. The substrates are used as an outer cover in disposable absorbent garments. As used herein, the term "print" or "printing" means to make an image on something. Frequently, printing involves formation of an image by the transfer of pigment, colorant, or brightener in the form of ink, wax, paint, or the like. The printed image is visible to the human eye and can includes, for example, shapes, patterns, designs, objects, likenesses of real or fictitious characters, or the like, or combinations thereof. For example, printing can include the direct transfer of pigment from a print roll, plate, or screen to the substrate. Printing can also include the deposit of pigment from a pigment source on a substrate without direct contact between the pigment source and the substrate, such as, for example from ink jet printers, wax jet printers, bubble jet printers, laser Jet printers, or the like, or combinations thereof. As used herein, the term "substrate" means a web of material capable of moving through a machine. A substrate can include contiguous material wherein individual units of material are connected or directly joined to the immediately preceding and trailing units of material. For example, a substrate may include a continuous web of woven material, nonwoven material, film, or the like, or combinations thereof. A substrate may also include interconnected absorbent articles in various stages of manufacture. A substrate may also include a web of discrete units of material separated by space or by other materials. For example, a substrate may include discreet absorbent articles moving through a converting operation via one or more conveyor belts or other means of conveyance known in the art.

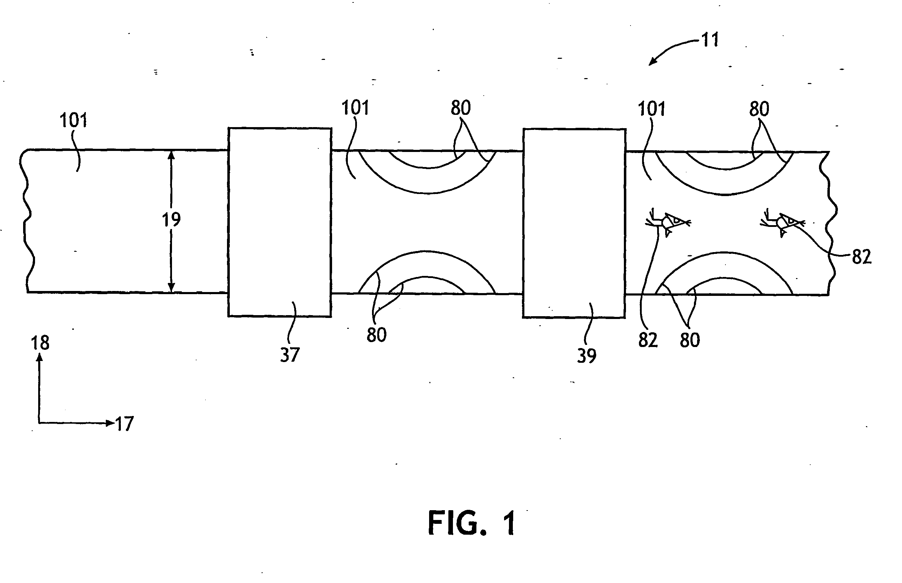

One embodiment of the method is generally illustrated at **11** in **Fig. 1****.** In **Fig. 1**, a moving substrate **101** is supplied to a first converting operation **37**. The substrate **101** has a machine direction **17**, a cross machine direction **18**, and a width **19**. The width **19** is measured in the cross machine direction **18**. The first converting operation **37** prints at least one first graphic **80** on the substrate **101.** The substrate **101** is then supplied to a second converting operation **39**. The second converting operation **39** prints at least one second graphic **82** on the substrate **101.** In various embodiments, the number of converting operations may be 3, 4, 5, 6, 7, 8, 9, 10, or more than 10. In various embodiments, the substrate **101** may be slit and/or wound and then transported to the second converting operation **39** wherein the substrate **101** is unwound or otherwise delivered to the second converting operation **39**.

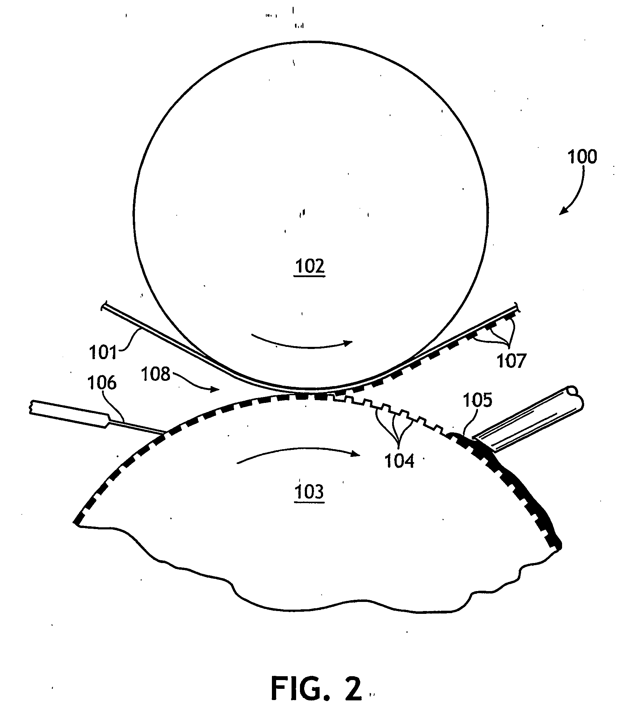

The first converting operation **37** is a traditional contact printing process. Contact printing has traditionally been an economical way to print large and/or wide patterns on a substrate with reproducible results. However, contact printing may result in higher initial expense because the specific pattern rolls, plates, or screens must be manufactured. Because of the custom manufacturing, subsequent changes to the print pattern may require the manufacture of new equipment and may limit the economic feasibility of quickly changing the print pattern for specific needs or limited production situations. As used herein, "contact printing" refers to a form of printing in which the substrate is directly contacted by the printing apparatus. For example, the first converting operation **37** may include a gravure process as generally illustrated at **100** in **Fig. 2**. Printing via a gravure process would be an example of contact printing. In the gravure process **100**, a substrate **101** is passed between a rubber impression roller **102** and a gravure cylinder **103**. The surface of the gravure cylinder contains a large number of depressions or cells **104**, which are designed to receive, hold, and transfer colorant to the substrate **101**. Colorant or brightener **105** is applied to the surface of the gravure cylinder **103** downstream of a nip **108** and is removed from the land areas of the gravure cylinder with a doctor blade **106.** As the substrate **101** enters the nip **108,** it is pressed against the gravure cylinder **103** by the rubber impression roller **102**, thereby permitting the colorant or brightener **105** to transfer from the gravure cylinder cells **104** and be deposited on the surface of the substrate **101** in small colored or brightened areas **107** corresponding to the individual gravure cylinder celts **104**. When printing with inks, the overall pattern of small colored or brightened areas remains relatively intact in the final substrate. In such instances, the percentage of the surface area of the substrate **101** covered by the colored areas will closely match the percentage of the surface area of the gravure roll covered by the gravure cells. The gravure process **100** uses an engraved print roll **103** that increases the life of the print pattern and provides higher definition when printing on plastic substrates. Additionally, gravure equipment can be used with water-based, solvent-based, and hot-melt, adhesive-based inks, or the like.

The first convertihg operation **37** may alternatively comprise flexographic printing as is well known in the art. Flexographic printing provides high speed, high quality printing suitable for printing nonwoven fibrous webs, while maintaining the tactile softness of the web. Flexography is a printing technology, which uses flexible raised rubber or photopolymer plates to carry the image to a given substrate. The flexible plates carry a typically low-viscosity ink directly onto the substrate. The quality of flexographic print in recent years has rapidly advanced such that, for many end-uses, it is comparable to lithographic or gravure printing.

The types of plates that can be used with the process include, but are not limited to, plates identified as DuPont Cyrel.RTM.HL, PQS, HOS, PLS, and LP, which may be obtained from E. I. DuPont de Nemours & Co., Inc., 1007 Market Street, Wilmington, Delaware 19898, U.S.A.; a plate identified as BASF Nyloflex.RTM., which may be obtained from BASF, 1255 Broad Street, Clifton, New Jersey 07015, U.S.A.; and a plate identified as Flex-light. RTM. type FL-SKOR.RTM., which may be obtained from W.R. Grace & Co., 5210 Phillip Lee Drive, Atlanta, Georgia 30336, U.S.A. Others include laser etched vulcanized rubber cylinders, such as those supplied by Luminite Products Corporation, 115 Rochester Street, Salamanca, New York 14779, U.S.A. or by Flexo Empress, 270 Rochester Street, Salamanca, New York 14779, U.S.A,; or rubber printing plates, such as those supplied by Fulflex, Incorporated, P.O. Box 4549, Middleton, Rhode Island 02804, U.S.A. The rubber plates and vulcanized cylinder could be natural rubber, EPDM, nitrites, or urethanes.

The first converting operation **37** may alternatively comprise lithographic printing, offset printing, or screen printing as is well known in the art. Lithographic printing is based on the immiscibility of oil and water. Ink receptive areas are generated on the surface of a hydrophilic surface. When the surface is moistened with water and then ink is applied, the hydrophilic background areas retain the water and repel the ink and the ink receptive areas accept the ink and repel the water. The ink is transferred to the surface of a material upon which the Image is to be reproduced. Typically, the ink is first transferred to an intermediate blanket, which in tum transfers the ink to the surface of the material upon which the image is to be reproduced. The screen printing process typically forces ink through unblocked areas of a metal, synthetic or silk fiber screen by spreading the ink onto the screen and passing a squeegee over the screen thus forcing the ink onto the substrate. Offset printing typically involves a transfer roll that is capable of being rotated. An ink source is located proximate to the transfer roll and dispenses ink onto the transfer roll. A web is present and contacts the transfer roll. The substance is dispensed onto the transfer roll and is transferred to the web through contact of the transfer roll and the web.

The second converting operation 39 includes a manufacturing line for absorbent garments, such as, for example, a diaper converting operation. As such, the second converting operation 39 may utilize a non-contact ink jet printing process to print at least one second graphic 82 on the previously printed substrate 101. Use of ink jet printing is well suited to producing fine patterns with high detail. Ink jet printing also allows rapid changes to the pattern with only a change in programming versus fabrication of new printing rolls, screens, or plates. However, non-contact printing may not be optimum for producing wide patterns or patterns requiring a large quantity of ink expenditure. As used herein, "non-contact printing" refers to a form of printing in which an image is formed on a substrate without direct contact between the substrate and the apparatus producing the image.

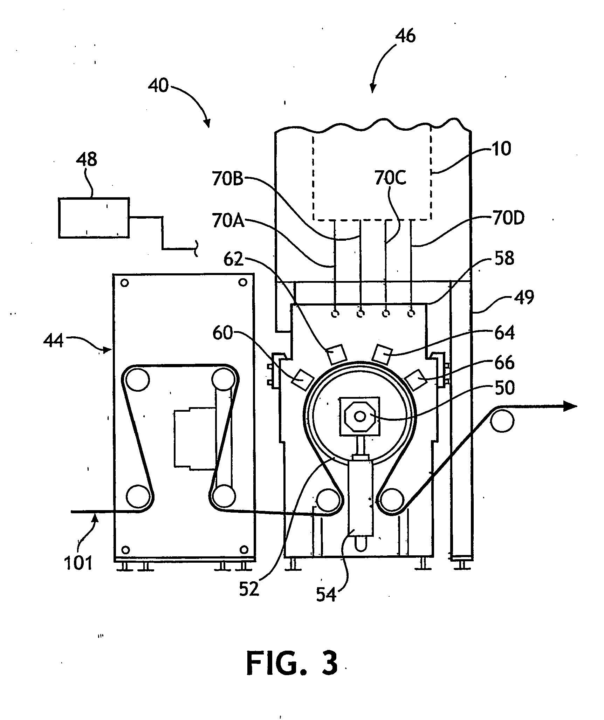

As an example of non-contacting printing, the second converting operation **39** may include an ink jet printing process as generally illustrated at **46** in **Fig. 3**. The Ink jet process **46** includes apparatus **40** that may be used for printing at least one second graphic **82** on substrate **101**. The substrate **101** is used as an outer cover. The substrate **101** may be a complete or partially complete diaper or other absorbent garment. The substrate **101** will be illustrated as an outer cover for an absorbent garment, such as a diaper. Thus, the second graphic **82** may be formed in the same process as the assembly of the various components of the diaper. However, in particular embodiments, the first and second converting operation can include printing and not the assembly of an absorbent garment. The composition and configuration of diapers and diaper converting operations are well understood by those of ordinary skill in the art and are representatively disclosed by way of illustration in U.S. Patent 5,743,994 issued April 28, 1898 to Roessler et al., U.S. Patent 5,827,387 issued October 27, 1998 to Reynolds et al., U.S. Patent 5,827,259 issued October 27, 1998 to Lax et al., and U.S. Patent 5,853, 402 issued December 29, 1998 to Faulks et al. Referring to **Fig. 3****,** the outer cover material may be fed from a roll (not shown) as a substrate **101** to a guide **44**, then to an ink jet printing station **46** and thereafter to the assembly line (not shown). A controller **48** can be used to control the operation of the apparatus **40**, and in particular the operation of the ink jet printing station **46**. In a particular aspect, a web guide **44** can be used to monitor the cross machine direction position of the substrate 101 to maintain it in a controlled position just prior to entering the printing station **46**. An example of a suitable web guide is the Symat 50 Offset Pivot Guide available from Fife Corporation of Oklahoma City, Oklahoma, U.S.A.

The illustrated ink jet printing station **46** includes a frame **49** which supports bearings 50 (only one is shown) holding a print drum **52** for rotation about a generally horizontal axis. A suitable bearing would be a model number F4BDL200 manufactured by Dodge Bearings Incorporated of Greenville, South Carolina, U.S.A. These bearings **50** provide resistance to vibration of the drum **52**, which is particularly likely to occur during start up and shut down of the diaper assembly apparatus. Vibrations can cause the Image to appear fuzzy to the point that diapers may have to be discarded as commercially unacceptable in the absence of vibration control. The bearings **50** supporting the print drum **52** for rotation can be mounted for slidingly adjustable vertical movement in the frame **49**. Two electrically driven linear motion actuators **54** (only one is shown), on opposite sides of the frame **49**, can be connected to the print drum bearings **50** for moving the bearings and the print drum **52** relative to the frame. A suitable linear actuator is a Model GSX30 manufactured by Exlar Corporation of Chanhassen, Minnesota, U.S.A.

An on demand piezoelectric ink jet printing unit may include a housing **58** mounted on the frame **49**. The illustrated housing **58** supports first through fourth print heads (designated **60, 62, 64**, **66,** respectively), arranged along an upper portion of the circumference of the print drum **52** although any number of print heads may be used. The print heads **60-66** are located in a series along the circumference portion. The lower ends of the print heads **60-66** (from which the ink droplets are discharged) may be spaced closely with the drum **52**. As an example, the print heads **60-66** can be spaced from the drum **52** so that the spacing between the upper surface of the substrate **101** and the print heads is between about 1 mm (0.04 In) and about 3 mm (0.12 in). In one embodiment, the cylinders **54** move the print drum **52** so that in operation, the spacing of each print head **60-66** from the web Is about 1 mm (0.04 in) to 3 mm (0.12 in) in operation, and is larger when not in use for facilitating threading the substrate **101** material through the ink jet printing apparatus 46. Movement is accomplished by the linear actuators **54**.

Although other inks could be used to print the second graphic **82**, it has been found that thermochromic or phase change inks, such as wax-based inks have certain advantages in that these inks have reduced tendencies to spread and smear and do not require time or equipment for drying, which is highly desirable in a manufacturing context. These types of inks also do not require the use of solvents. Wax-based inks are received in solid blocks that are melted in an ink supply system 70, schematically illustrated as being mounted on the frame 49. The melted ink of a particular color is fed through a respective one of four lines (designated **70A, 70B, 70C**, **70D.** respectively) to one of the print heads **60, 62, 64, 66,** as demanded by the print head. One suitable system for melting and supplying the ink is the DYNAMELT® S Series hot melt supply system available from ITW Dynatec of Hendersonville, Tennessee. U.S.A. This system, marketed for supplying adhesives, can be modified for supplying melted ink. In one embodiment, the system includes a metered gear pump (not shown) for precise delivery of ink, which gear pump includes stainless steel components in contact with the ink. Moreover, the commercially available system can be further modified by adding a 10 micron filter. Other filter sizes may be used as specified by the print head vendor or ink vendor. The ink supply system controls the temperature of the ink. In one embodiment, the melted ink is maintained at about 125 °F (51.7 °C). The temperature may be controlled to any level specified by the ink vendors or the print head vendors.

Suitable print heads **60, 62**, **64, 66** that may be used in one embodiment to produce an image are Galaxy PH 256/80 HM 256 channel ink jet print heads (Serial Nos. 5601320, 5601325, 5601326, 5601327) available from Spectra, Inc. of Lebanon, New Hampshire, U.S.A. These print heads **60-66** each have two piezoelectric crystals with each crystal having two independent electrical circuits. Each circuit is associated with **64** orifices for a total of 256 orifices per print head. Each of the orifices can be individually addressed so that the controller **48** can select which of the 256 orifices are to be fired in each cycle. The orifices are spaced apart about 0.0254 centimeter (0.01 inch) ± 0.00245 centimeter (0.001 inch). It will be understood that other print heads having a different number of orifices and/or orifices with different spacings could be used without departing from the scope of the present invention. A longer print head would allow a wider (in the cross direction of the outer cover material) image to be printed. Moreover, print heads may be banked (placed side-by-side in the cross direction) to print a wider image. The print heads **60-66** can each have an ink reservoir on the print head that has an ink level sensor that can be used to signal the ink supply system 70 to deliver additional ink to the print head.

Application of ink by ink jet print heads to high speed substrates is also discussed in Sharma et al., U.S. Patent application No. 10/330,575, entitled HIGH-SPEED INK JET PRINTING FOR VIBRANT AND CROCKFAST GRAPHICS ON WEB MATERIALS OR END-PRODUCTS, filed December 27, 2002. Other suitable apparatus, ink types, color measurements, and design techniques are also discussed in Anderson et al, U.S. Patent Application No. 10/623,030, entitled ABSORBENT ARTICLE WITH HIGH QUALITY INK JET IMAGE PRODUCED AT LINE SPEED, filed July 18, 2003.

In various embodiments, the substrate 101 may be moving at least 100 feet per minute (fpm) during the non-contact printing operation 46 in the second converting operation 39. Alternatively, the substrate 101 may be moving at least 200 fpm², 300 fpm², 400 fpm², 500 fpm², 600 fpm², 700 fpm², 800 fpm². 900 fpm². or 1000 fpm² during the non-contact printing operation 46 in the second converting operation 39. In various other embodiments, the substrate 101 may be moving faster than 1000 fpm ²during the non-contact printing operation 46 in the second converting operation 39.

In various embodiments, the first converting operation 37 may include multiple contact printing steps wherein one or more colors or patterns are applied.

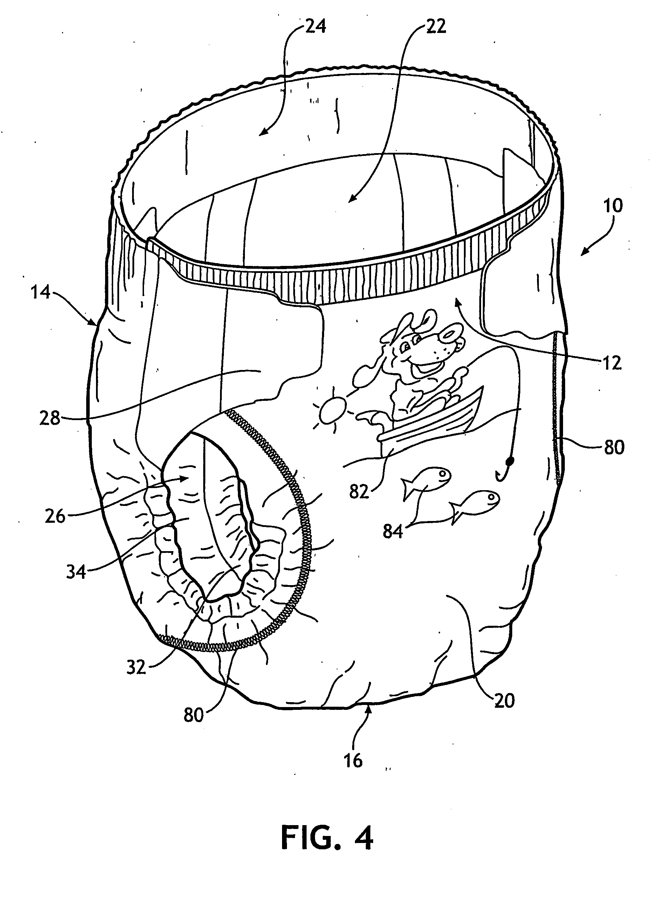

Referring now to **Fig.** 4, an absorbent garment in the form of a diaper (indicated generally at 10) having at least one first image **80** and at least one second image **82** may be formed by the method of the present invention. The diaper **10** comprises a front waist region **12**, a back waist region **14.** and an intermediate crotch region **16** interconnecting the front and back waist regions. The waist regions **12** and **14** comprise those portions of the diaper **10** which when worn, wholly or partially cover or encircle the waist or mid-lower torso of the wearer. The intermediate crotch region **16** lies between and interconnects the waist regions **12** and **14**, and comprises that portion of the articles **10** which, when worn, is positioned between the legs of the wearer (not shown) and covers the lower torso of the wearer. The exterior of the diaper **10** is formed substantially by an outer cover 20 constructed to form a liquid barrier.

The diaper 10 has a generally three-dimensional configuration, as illustrated in Fig. 4, when fastened on a wearer. In this configuration the diaper has an interior space 22 for receiving the lower torso of a person wearing the diaper, a waist opening 24 for receiving the wearer into the interior space of the diaper, and a pair of leg openings 26 (only one is shown). In the illustrated embodiments, the fastener tabs 28 are permanently attached to a back waist region 14 of the diaper. These tabs 28 may be releasably attached to the front waist region 12 of the diaper 10 for securing the diaper around the lower torso of the wearer. Other fastening systems (not shown) may be employed without departing from the scope of the present invention.

The diaper **10** generally includes a bodyside liner 32 on its innermost surface that contacts the skin of the person wearing the diaper 10. The liner 32 is desirably a soft, compliant material which is highly liquid permeable and hydrophobic to permit passage of liquid through the liner and to maintain a relatively dry surface contacting the skin. These types of materials are well known to those of ordinary skill in the art and these need not be more fully described herein. Between the bodyside liner 32 and the outer cover 20, there is typically some form of liquid retention structure (not shown), such as an absorbent pad made of fibrous absorbent material and superabsorbent material (SAM). An example of such an absorbent pad is shown in co-assigned U.S. Patent No. 6,383,960. There is also typically a surge layer (not shown) of material that rapidly absorbs liquid passing through the bodyside liner. The surge layer can distribute the liquid over a larger surface area before releasing It to the absorbent pad. Suitable surge layers are described in co-assigned U.S. Patent Nos. 4,798,603, 6,364,382, 5,429,829, 5,490,846, 5,522,810 and 5,562,650.

To further enhance containment and/or absorption of body exudates, the article **10** may include a front waist elastic member, a back waist elastic member, and leg elastic members, as are known to those skilled in the art. The waist elastic members can be operatively joined to the outer cover **20** and/or body side liner **32** along opposite waist edges, and can extend over part or all of the waist edges. The leg elastic members are desirably operatively joined to the outer cover **20** and/or body side liner **32** along opposite side edges of the diaper **10** and positioned in the crotch region 16 of the diaper.

The waist elastic members and the leg elastic members can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and attached to a substrate, attached to a gathered substrate, or attached to a substrate and then elasticized or shrunk, for example with the application of heat; such that elastic constrictive forces are imparted to the substrate. In one particular embodiment, for example, the leg elastic members include a plurality of dry-spun coalesced multifilament spandex elastomeric threads sold under the trade name LYCRA® and available from E.I. DuPont de Nemours and Company, Wilmington, Delaware. U.S.A.

To still further enhance containment and/or absorption of any body exudates discharged from the wearer, the diaper **10** may include a pair of containment flaps **34** (only one is shown) which are configured to provide a barrier to the transverse flow of body exudates. A flap elastic member (not shown) may be operatively joined with each containment flap **34** in any suitable manner as is well known in the art. The elasticized containment flaps **34** define an unattached edge that assumes an upright, generally perpendicular configuration In at least the crotch region **16** of the diaper **10** to form a seal against the wearer's body. The containment flaps **34** can be located along the transversely opposed side edges of the diaper 10, and can extend longitudinally along substantially the entire length of the diaper or may only extend partially along the length of the diaper. Suitable constructions and arrangements for the containment flaps 34 are generally well known to those skilled in the art.

The outer cover **20** desirably comprises a material which is substantially liquid impermeable, and can be elastic, extensible or nonstretchable. The outer cover **20** can be a single layer of liquid impermeable material, but desirably comprises a multi-layered laminate structure in which at least one of the layers is liquid impermeable. For instance, the outer cover **20** can include a liquid permeable outer layer and a liquid impermeable inner layer that are suitably joined together by a laminate adhesive, ultrasonic bonds, thermal bonds, or the like. Suitable laminate adhesives, which can be applied continuously or intermittently as beads, a spray, parallel swiris, or the like, can be obtained from Findley Adhesives. Inc., of Wauwatosa, Wisconsin, U.S.A., or from National Starch and Chemical Company, Bridgewater, New Jersey U.S.A. The liquid permeable outer layer can be any suitable material and is desirably one that provides a generally cloth-like texture. One example of such a material is a **20** gsm (grams per square meter) spunbond polypropylene nonwoven web. The outer layer may also be made of the same materials as the liquid permeable bodyside liner **32**. While it is not a necessity for the outer layer of the outer cover **20** to be liquid permeable, it is desired that it provides a relatively cloth-like texture to the wearer. The outer layer may generally be any suitable nonwoven material as is known in the art.

The inner layer (not shown) of the outer cover **20** can be both liquid and vapor impermeable, or it may be liquid impermeable and vapor permeable. The inner layer can be manufactured from a thin plastic film, although other flexible liquid impermeable materials may also be used. The inner layer, or the liquid impermeable outer cover when a single layer, prevents waste material from wetting articles, such as bed sheets and clothing, as well as the wearer and caregiver. A suitable liquid impermeable film for use as a liquid impermeable Inner layer, or a single layer liquid impermeable outer cover 20, is a 0.02 millimeter (0.000787 inches) polyethylene film commercially available from Pliant Corporation of Schaumburg. Illinois, U.S.A. Alternatively, a suitable liquid impermeable film is a highly breathable film made primarily of polyethylene and calcium carbonate having a basis weight of 19 grams per square meter and a thickness of 0.02 millimeter (0.000787 inches). Such a film is commercially available from Pliant Corporation of Schaumburg. Illinois, U.S.A. and is designated Type XP-8635Y.

If the outer cover is a single layer of material, it can be embossed and/or matte finished to provide a more cloth-like appearance. As earlier mentioned, the liquid impermeable material can permit vapors to escape from the interior of the disposable absorbent article, while still preventing liquids from passing through the outer cover. A suitable "breathable" material is composed of a microporous polymer film or a nonwoven fabric that has been coated or otherwise treated to impart a desired level of liquid impermeability. As used herein, the term "nonwoven fabric" refers to a fabric that has a structure of individual fibers or filaments which are interlaid, but not in an identifiable repeating manner. Nonwoven fabrics have been, in the past, formed by a variety of processes known to those skilled in the art such as, for example, meltblowing, spunbonding, wet-forming and various bonded carded web processes. A suitable microporous film is a PMP-1 film material commercially available from Mitsui Toatsu Chemicals, Inc., Tokyo, Japan, or an XKO-8044 polyolefin film commercially available from 3M Company, Minneapolis, Minnesota U.S.A. A description of alternative outer cover materials made of extensible materials (so that the outer covers are expandable) can be found in co-assigned U.S. Patent No. 6,264,641, entitled EXPANDABLE COVER GARMENT, issued July 24, 2001.

In various embodiments, the substrate **101** may be an outer cover **20** of a diaper **10.** In the illustrated embodiment of **Fig, 4**, the diaper **10** has a first graphic **80**, a second graphic **82**, and a third graphic **84** applied in a front waist region **12** of the outer layer of the outer cover **20** of the diaper **10**. In various embodiments, the outer cover **20** may provide a white background on which the graphics **80, 82**, or **84** are applied or the background could be other, non-white colors selected for making colors in the graphic stand out. In various embodiments, wherein the outer cover **20** has more than one layer, the graphics **80, 82**, and **84** may be applied on an inner layer of the outer cover **20**, an outer layer of the outer cover **20**, or both. The graphics **80**, **82.** and **84** may be applied to either side of the layers of the outer cover **20**. For example, some, all, or none of the graphics **80, 82**, or **84** may be "between" the inner and outer layers of the outer cover **20** after lamination. In a suitable embodiment, the first graphic **80** is applied to an inner layer of an outer cover **20** and the second graphic **82** is applied to an outer layer of the outer cover **20.**

In various embodiments, the inner layer and outer layers of the outer cover **20** may be laminated together before, after, or simultaneously with the application of the first graphic **80,** the second graphic **82**, the third graphic **84**, or combinations thereof. In various embodiments, the first graphic **80** may be applied on the same side of the substrate **101** as the second graphic **82**, third graphic **84**, or both. Alternatively, the first graphic **80** may be applied on the opposite side of the substrate **101** as the second graphic **82**, third graphic **84**, or both. Alternatively, the first graphic **80,** the second graphic **82**, the third graphic **84**, or combinations thereof may be on both sides of the substrate **101.** As used herein, the term "applied on" Includes those situations wherein the first graphic 80, second graphic 82, third graphic **84,** and/or additional graphics are applied to a first layer of an outer cover 20 and the pigment, ink, colorant, wax, or the like partially or completely penetrates the first layer and deposits partially or completely on the second layer. For example, the pigment, ink, colorant, wax, or the like may be applied on the nonwoven layer of an outer cover 20. The pigment, ink, colorant, wax, or the like may partially or completely penetrate the nonwoven layer and contact the film layer. In such case, the pigment, ink, colorant, wax, or the like would be "applied on" the nonwoven layer even though a portion ultimately was deposited on the film layer. The pigment, ink, colorant, wax, or the like would not be "applied on" the film layer in this example.

The substrate 101 is used as a component of an absorbent garment, and the first graphic 80 may be printed prior to formation of the absorbent garment, simultaneously with the formation of the absorbent garment, or after the formation of the absorbent garment. Similarly, the second graphic 82 and/or third graphic **84** may be printed prior to formation of the absorbent garment, simultaneously with the formation of the absorbent garment, or after the formation of the absorbent garment.

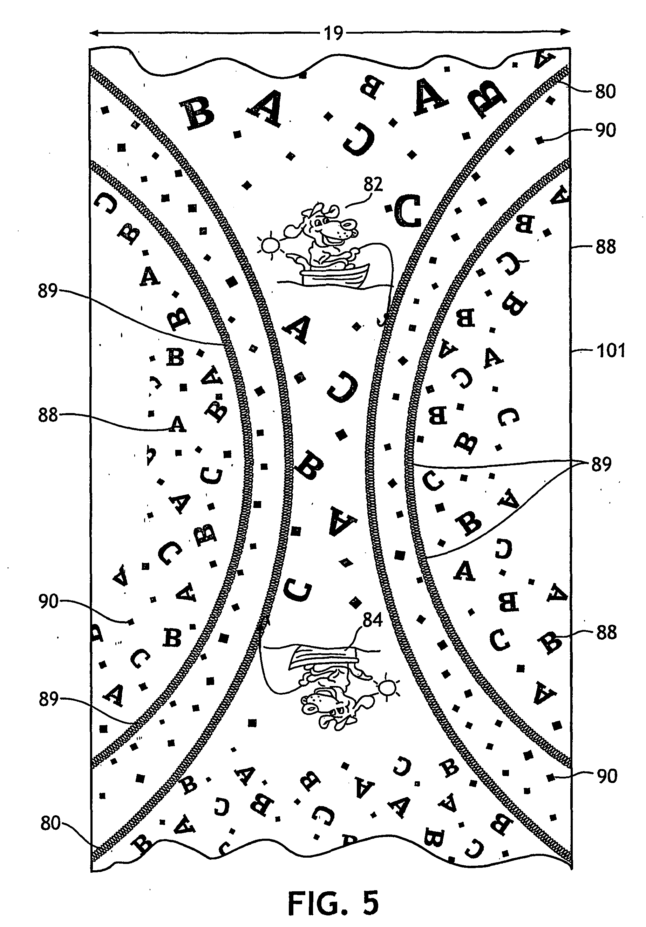

Referring now to Fig. **5****,** a portion of an exemplary substrate **101,** suitable as an outer cover of a diaper, is illustrated. The substrate 101 has a first graphic 80, a second graphic 82, and a third graphic **84.** In various embodiments, the first graphic 80 may be a background graphic. As used herein, the term "background graphic" means an image, including components of an image, which provides the scenery or backdrop behind one or more images, such as, for example, a feature graphic. The background graphics can include the part of an image representing what lies behind the image or images in the foreground. Additionally, background graphics can provide a less conspicuous image as compared with the feature graphic. As used herein, the term "feature graphics" refers to graphics by which the product may be easily recognized. Typically, such graphics would constitute the "focus" of the visual markings and generally would consist of greater detail, sharpness, and/or color than those used for background graphics. However, in various embodiments, the background graphics and feature graphics may be equally intense and attractive to a consumer. For example, a first graphic **80**, as illustrated in **Fig. 5**, is as vibrant and attractive as a second graphic **82** and a third graphic **84**, but the first graphic **80** could be considered a background graphic, whereas the second graphic **82** and/or third graphic **84** could be considered feature graphics. In some embodiments, the feature graphic or graphics may include, for example, cartoon characters, animals, cartoon animals, vehicles, toys, flowers, numbers, letters, or the like, or combinations thereof.

In various embodiments, the first graphic **80** may span 100% of the width **19** of the substrate **101** as illustrated in Figs. **5****,** **6** and **7****.** As used herein, the term "span" means to extend from one edge to another edge. As seen in **Figs. 5****,** **6** and **7****,** the first graphic **80** reaches from one edge of the substrate **101** to the other edge of the substrate **101** thus spanning 100% of the width **19** of the substrate **101**. The unprinted spaces within the first graphic **80** do not change the span of the graphic. In alternative embodiments, the first graphic **80** may span at least 90%. 80%. 70% or 60% of the width **19** of the substrate **101**. In the illustrated embodiment of **Fig. 5**, the first graphic **80** comprises subcomponents **88, 89**, and **90**. The subcomponent **88** includes the letters "A, B, C." The subcomponent **89** includes four curved lines. The subcomponent **90** includes diamond-shaped objects. The first graphic **80** is applied during a first converting operation **37**. In various embodiments, the second graphic **82**, the third graphic **84,** or additional graphics may be generally centered along the width **19** of the substrate **101** as illustrated In Figs. **5**, **6****,** and **7**. The second graphic **82**, the third graphic **84,** or additional graphics may be printed within the center 1/3 of the width **19** of the substrate **101**.

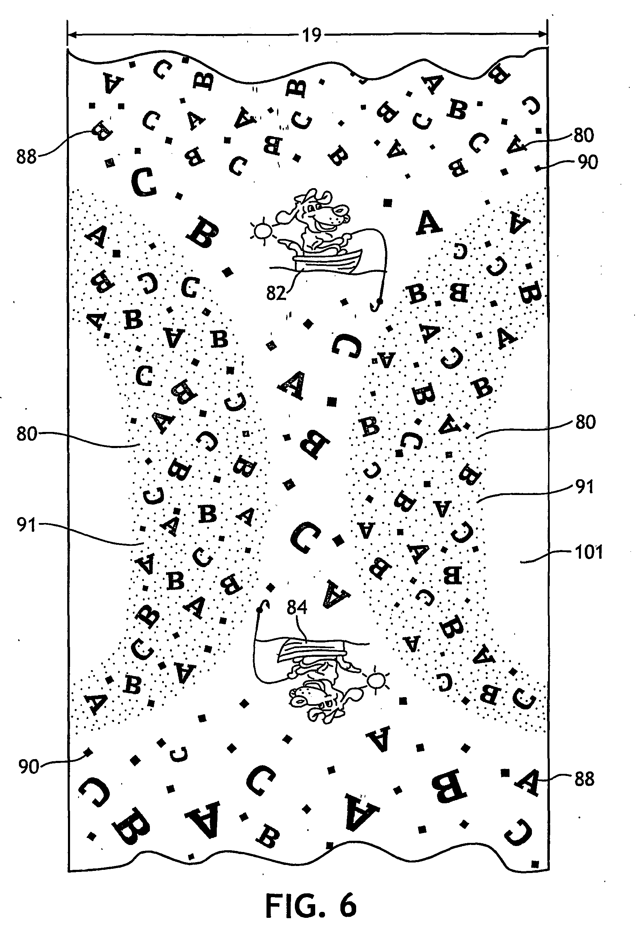

The substrate **101** of Fig. **5** is suitable for use as an outer cover In an absorbent garment, such as the outer cover **20** of the diaper **10,** as illustrated In **Fig. 4**. Referring specifically to the embodiments Illustrated in **Figs. 5** and **6**, the second graphic **82** includes a dog fishing. The third graphic **84** includes the same dog fishing but with the orientation reversed. This orientation of the second graphic **82** and the third graphic **84** is advantageous when manufacturing an absorbent garment. For example, the second graphic **82** may be registered such that the second graphic **82** is positioned in the front waist region **12** of an absorbent garment **10**, such as that illustrated in **Fig. 4**. Additionally, the third graphic **84** may be registered such that the third graphic **84** is positioned in the back waist region **14** of an absorbent garment **10.** As such, both the second graphic **82** and the third graphic **84** would be positioned in a "heads up" configuration in the fully assembled diaper. In this embodiment, the second graphic **82** and the third graphic **84** are examples of feature graphics. Similarly, the first graphic **80** may be registered such that subcomponent **89** is centered generally in the crotch region **16** of an absorbent garment **10.** As such, the subcomponent **89** of first graphic **80** may be registered relative to the leg openings **26**.

Referring now to Fig. **6****,** a portion of a second exemplary substrate **101,** suitable for use as an outer cover for an absorbent garment, is illustrated. The substrate **101** has a first graphic **80**, a second graphic **82**, and a third graphic **84** similar to the embodiment illustrated in **Fig, 5****.** However, in **Fig. 6**, the first graphic **80** comprises subcomponents **88, 90**, and **91**. Subcomponent **89.** curved lines, has been replaced by subcomponent **91**, which includes a curved shadow. In a suitable embodiment, the first graphic **80** may be registered such that subcomponent **91** is centered generally in the crotch region 16 of an absorbent garment **10**. As such, the subcomponent **91** of first graphic **80** may be registered relative to the leg openings **26**.

In various embodiments, the graphics may be comprised of different colors. For example, in some embodiments, the first graphic **80** may be comprised of 1 color, 2 colors, 3 colors, 4 colors, 5 colors, 6 colors, 7 colors, 8 colors, 9 colors, 10 colors. 11 colors, or 12 colors. In other embodiments, the first graphic **80** may be comprised of more than 12 colors. In various embodiments, the second graphic **82** may be comprised of 1 color, 2 colors, 3 colors, 4 colors, 5 colors, 6 colors, 7 colors, 8 colors, 9 colors, 10 colors, 11 colors, or 12 colors. In other embodiments, the second graphic 82 may be comprised of more than 12 colors. In various embodiments, the third graphic **84** may be comprised of 1 color, 2 colors, 3 colors, 4 colors, 5, colors, 6 colors. 7 colors, 8 colors, 9 colors, 10 colors, 11 colors, or 12 colors. In other embodiments, the third graphic **84** may be comprised of more than 12 colors. In various other embodiments, more than 3 graphics may be applied.

The present methods may include non-contact printing means with the capability to change the second graphic **82** and/or third graphic **84** without purchasing or manufacturing new equipment, such as a printing roll, plate, or screen. For example, controllers associated with ink jet printing, bubble jet printing, wax jet printing, laser jet printing, and the like, and combinations thereof, can be programmed to change the second graphic **82**, the third graphic **84**, and/or additional graphics as desired. For example, the graphics may be part of a story line, may be randomly selected, may be seasonal, may be customized, or the like, or combinations thereof.

As used herein, the term "story line" refers to multiple graphics that when taken as a whole convey a uniform and coherent story or theme. An example of a coherent story may comprise a nursery rhyme wherein the parts of the nursery rhyme are illustrated in sequential graphics applied over sequential absorbent products made by particular methods of the present invention. Alternatively, a story line may include a common background graphic with differing feature graphics all relating to the same theme. In one embodiment, the background graphic includes a pond, shore, and sky. The feature graphics associated with this background graphic may include a boat, people and/or cartoon characters in the boat, fish, people and/or cartoon characters fishing, people and/or cartoon characters swimming or playing on the shore, and the like, and combinations thereof. In this embodiment, the theme is "things associated with a pond." In another exemplary embodiment, the background graphic may include a flower patch and sky. The feature graphics associated with this background graphic may include people and/or cartoon characters planting seeds and watering. The feature graphics in this story line may also include the flowers in various stages of development, or may depict people and/or cartoon characters playing amongst the flowers, picking the flowers, or the like, and combinations thereof. In this embodiment, the theme is "things associated with a flower patch."

As used herein, the term "randomly selected" refers to the process of using a controller and a means of generating a random number to select graphics from a list of pre-selected graphics. For example, a background graphic, suitable for use with randomly selected graphics, may include a prairie field and sky. The feature graphics associated with this background, may be randomly selected from graphics of birds, bees, insects, clouds, sunshine, rain, animals, trees, trees with various fruits, flowers, flowers in various stages of development, paths, babies playing and exploring, and the like, and combinations thereof.

As used herein, the term "seasonal" refers to graphics that are changed so as to coordinate with the season and/or a particular region. For example, during the winter the graphics may, for example, depict snow, snow people, sleds, people and/or cartoon characters skiing or ice skating, or the like, or combinations thereof. During the fall, the graphics may depict, for example, leaves changing colors, fallen leaves, piles of leaves, pumpkins, com stalk bundles, and the like, and combinations thereof. Spring graphics may include, for example, blooming plants, flowers, trees, kites, and the like, and combinations thereof. Summer graphics may include, for example, people and/or cartoon characters swimming, boating, fishing, playing, or the like, or combinations thereof. Additionally, summer graphics may include baseballs, bats, soccer balls, bicycles, and the like, and combinations thereof. One skirted in the art will appreciate that other graphics are possible and the examples illustrated herein are illustrative and not meant to limit the scope of the present invention.

As used herein, the term "customized" refers to graphics that are changed to suit a small demographic, region, customer, or the like. For example, a common background graphic could be utilized for printing outer covers for absorbent articles. The feature graphics could be changed to provide a corporate logo for a limited production run of products for a particular retailer and then changed again to provide a different logo or motif for a second retailer. Additionally, the feature graphics may include various languages that would allow precise customization of products in small markets and/or regions. In such situations, the economy of scale realized when producing a consistent background graphic via contact printing is supplemented by customizing the feature graphics via non-contact printing to satisfy smaller demographics.

The present invention may utilize the capability of non-contact printing to rapidly change the feature graphics while still maintaining the cost effective use of contact printing for background graphics. For example, the first graphic **80** may depict a "standard" background that could accent or supplement various second and third graphics **82** and **84.** In the presently illustrated embodiments, the subcomponents **88. 89, 90**, and **91** of first graphic **80** can provide a consistent background for second and third graphics **82** and **84.** These aspects therefore utilize the consistency of a common background pattern produced at lower costs through traditional contact printing methods while also maximizing the ability to customize the substrate **101** via non-contact printing.

In various embodiments, at least a portion of the second graphic **82** and/or the third graphic **84** overprints at least a portion of the first graphic **80**. As used herein, the term "overprint means to print a second image in the same general location as a first image. Overprinting a second image may obscure the first image or may compliment the first image. As used herein, the term 'compliment' means to fill up, complete, or make whole. For example, the first image may form part of a master graphic and the second image may add to, and potentially complete, the master graphic. A master graphic could comprise two or more images that combine to make a single image. As used herein, the term "master graphic" refers to a visual composite, which is built by individual images. For example, a first image may include a flower pot and a second image may overprint flowers in the flower pot. The master graphic would include the visual "whole" of a flower pot containing flowers. A second example of a master graphic includes a first image of a pond with at least one second image of fish overprinted. The master graphic would include the visual "whole" of fish swimming in a pond.

Another example of overprinting involves creating a first image that advertises the absence of a second image. If the second image overprints the first image, the advertisement of the absence is obscured and only the second image is seen. However, if the second image fails to overprint the first image, then the first image will remain visible.

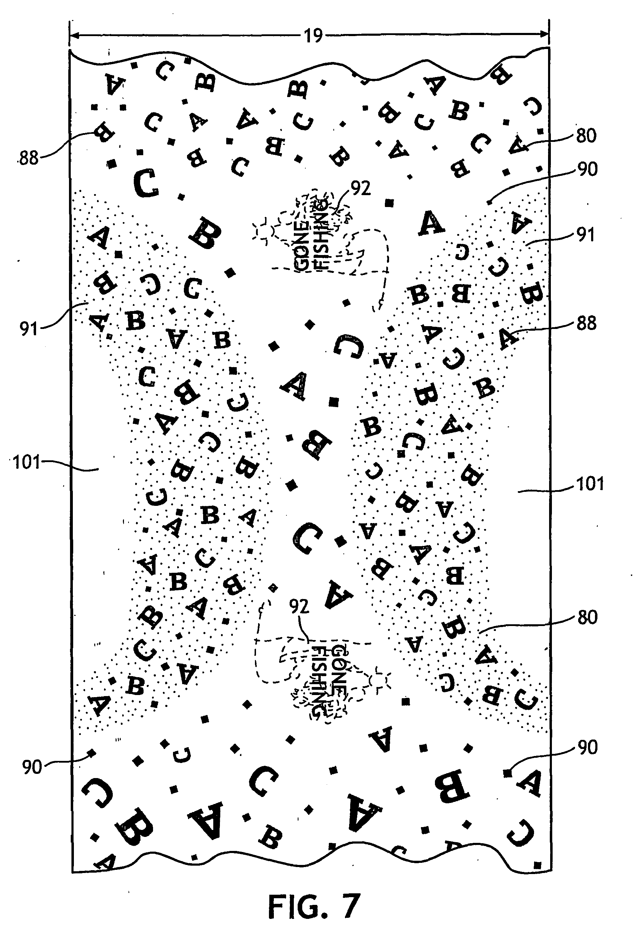

Referring now to **Fig. 7****,** a portion of an exemplary substrate **101**, suitable for use as an outer cover of an absorbent garment, is Illustrated. The substrate **101** includes a first graphic **80**. The first graphic **60** includes four subcomponents: **88, 90, 91**, and **92**.
Subcomponents **88, 90** and **91** are similar to those described in **Fig. 6** above. However, subcomponent **92** is an absence advertisement. As used herein, an "absence advertisement" is a graphic that conveys the message that an additional graphic is missing. The absence advertisements can take the form of pictures, words, outlines, shadows, or other forms of communication or combinations thereof. Failure to overprint the absence advertisement **92** does not result in a defective substrate or product because the absence advertisement **92** creates a "backup" system for printing. For example, if the second graphic **82** and/or the third graphic **84** overprints the absence advertisement **92**, the absence advertisement **92** is obscured and the overprinted graphic is visible in the finished substrate or product. However, if the overprinting fails, the absence advertisement **92** remains visible in the finished substrate or product. As a consequence, the finished substrate or product Is not wasted because the absence advertisement **92** is chosen such that the appearance of the absence advertisement **92** in the finished substrate or product would convey a desirable message, such as, for example, a humorous comment, contact information, coupon, or the like, or combinations thereof.

In various configurations, the absence advertisement can be used as a marketing device. For example, in Fig. 7, subcomponent 92 is an absence advertisement. As illustrated, subcomponent 92 includes the dotted outline of a dog, sitting in a bout, with a fishing pole and fishing line. Included within the dotted outline are the words "Gone Fishing!" The combination of the outline figure of the dog and the words within the outline convey the message that the fishing dog is missing, i.e., an absence advertisement. Alternatively, only the dotted outline may be used or only the words may be used. In various embodiments, a second graphic **82** could be overprinted on subcomponent **92**. If the overprinting was successful, only the second graphic **82** would be visible (see for example element 82 of **Fig. 6****)** as the absence advertisement Is at least partially obscured by the overprinted graphic. However, if the overprinting failed, the subcomponent **92** would be visible in the finished substrate or product, as illustrated in **Fig**. **7****.** Despite the printing failure, the resulting substrate and/or product would not be wasted because the absence advertisement **92** could be marketed as, for example, a game or contest. In the illustrated embodiments, a consumer would normally see the second graphic **82** as illustrated in **Fig. 6****,** but would on occasion see subcomponent **92** of the first graphic **80** as illustrated in **Fig, 7**. The absence advertisement **92** would include the consumer in any such "game" of the missing graphic, thus providing a backup system for printing and therefore reducing waste associated with printing failures. In **Fig. 7**, the consumer would be included in the search for the missing graphic when they viewed the absence graphic **92**. As such, instead of a defective product, the consumer would be notified that the second graphic 82 had "Gone Fishing1" thus turning what was typically considered a product failure into a product attribute.

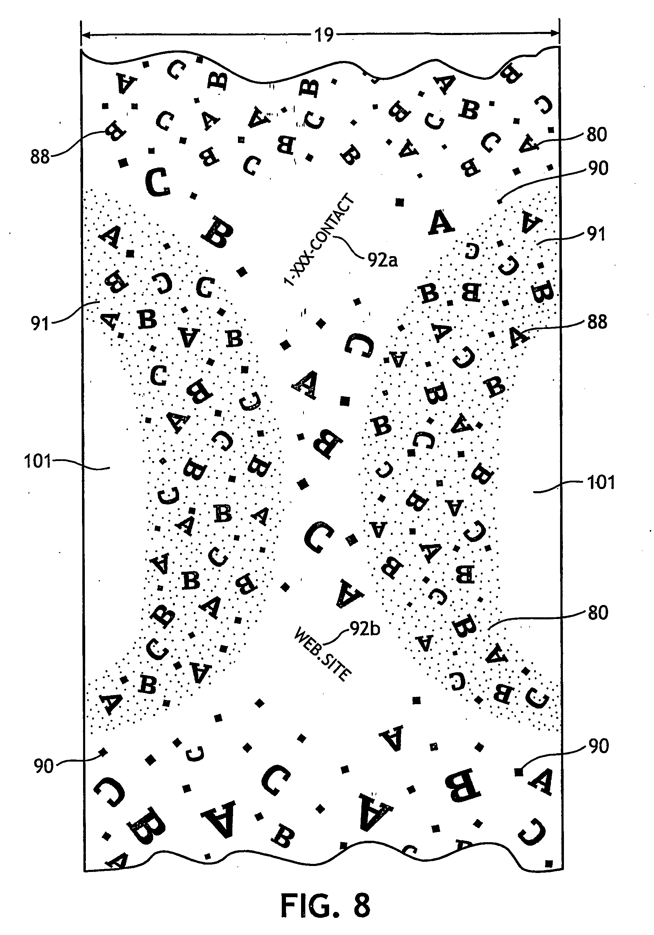

In an alternative embodiment, the absence advertisement **92** may include a consumer contact message. For example, referring now to **Fig. 8****,** a portion of an exemplary substrate **101,** suitable for use as an outer cover of an absorbent articles, is illustrated. The substrate **101** includes a first graphic **80.** The first graphic **80** includes four subcomponents: **88, 90, 91,** and **92.** Subcomponents **88, 90** and **91** are similar to those described in **Fig. 6** above. However, subcomponents **92a** and **92b** are examples of absence advertisements. The absence advertisement **92a** conveys a telephone number graphically depicted as "1-XXX-contact." Alternatively or additionally, the absence advertisement **92b** conveys a web site graphically depicted as "website." The absence advertisements **92a** and **92b** provide means of access to the customer service department, wherein, the consumer may register a complaint concerning the missing graphic and/or receive information concerning refunds or coupons. Alternatively, the absence advertisement 92a or **92b** may include coupon information associated with the telephone number or website. In such embodiments, the consumer could call or go online to receive a coupon or the like. Those skilled In the art will appreciate that many suitable absence advertisements are possible. Those included herein are illustrative and are not intended to limit the scope of the present invention.

Also disclosed is a method of distributing customized products to different customers. The method includes the steps of supplying a moving substrate to a first converting operation; contact printing at least one first graphic on the moving substrate, the first graphic being substantially uniform to all customers; supplying the moving substrate with the first graphic to a second converting operation; non-contact printing a plurality of second graphics on the moving substrate, the plurality of second graphics being customized for specific customers; and distributing the substrate to customers. As used herein, the term "substantially uniform" refers to a graphic or image that has generally the same appearance as another graphic or Image and the differences (If any) between the graphics or images are not noticeable to consumers without being directed to the differences.

The moving substrate with the first graphic may be divided into a plurality of rolls made of portions of the moving substrate prior to supplying the rolled substrate to at least one second converting operation. The moving substrate may be divided by shear cutting, slitting, or any other suitable severing processes as are well known in the art, or combinations thereof. Some rolls of substrate may be printed with one or more second graphics while others may be printed with a different second graphic. Some rolls may be printed with additional graphics in addition to the second graphics. In other arrangements, the plurality of rolls of substrate with only the first graphic may be sent to more than one second converting operation and may be printed with more than one second graphic. The second graphics may be different from one converting operation to another, within a particular converting operation, or both.

One method of distributing customized products to different customers includes the distribution of absorbent articles. The moving substrate may be an outer cover and the first converting operation may be a contact printing operation such as rotogravure printing, flexographic printing, offset printing, lithographic printing, screen printing, or the like, or combinations thereof. The first graphic may be printed on the outercover using one or more contact printing operations. The first graphic may be substantially uniform to all customers in a given group. A group may be formed based on ethnicity, nationality, location, interests, language, or any other desirable delineation or classification. For example, in **Fig. 5****,** the first graphic **80** may be printed on all outer covers for all customers in the group "North America." The outer cover may then be supplied with a substantially uniform graphic **80** to one or more second converting operations in one or more locations. In the present example, such converting operations may be one or more diaper converting operations where the outercover is combined with a liner and an absorbent to form a disposable diaper. During the diaper converting operation, one or more second graphics may be non-contact printed on the outer cover. The second graphics may be customized for specific customers. For example, customers within the group "North America" may include Canadian customers. United States of America customers, and Mexican customers. The second graphics may be customized by various non-contact printing methods as previously discussed. Customization may take the form of various languages. For example, the second graphics customized for the Canadian customers may include French and English text, the second graphics customized for the American customers may include English text, and the second graphics customized for the Mexican customers may include Spanish text. The disposable diaper with the substantially uniform graphics and the customized graphics may then be distributed to customers. Alternatively, the outer cover alone may be distributed to customers for the customers' use.

In other examples, the group may be the "world" and the customers may be various continents, countries, regions, or other desirable delineations. Alternatively, the customers may be specific businesses for which the customized graphic or graphics may include various indicia. As used herein, the term "indicia" refers to, for example, a company logo, company trademark, company phrase, licensed characters, proprietary characters, or other desirable marks, or combinations thereof, that are specific or desirable to a particular customer. In such embodiments, contact printing can produce substantially uniform graphics and non-contact printing can result In customized graphics prior to distribution to customers.

As discussed previously, the method of distributing customized products to different customers may include various substrates, including various combinations of laminates. Additionally, the graphics may include background graphics, feature graphics, complimentary graphics, master graphics, and absence advertisements. The graphics may also be part of a story line, may be randomly selected, may be seasonal, or may be customized.

In various embodiments, it may be desirable to align the various graphics through registration as is commonly known in the art. For example, the first graphic, second graphic, third graphic, or combinations thereof may be registered relative to the substrate, a registration mark, or any other graphic by means known in the art. For example, registration by use of a reference marker is taught in U.S. Patent 5,286,543 issued Feb. 15, 1994 to Ungpiyakul et al. Additionally, a process for registering two continuously moving layers by use of registration marks is taught in U.S. Patent 5,766,389 issued June 16, 1998 to Brandon et al.

While the invention has been described in detail with respect to specific embodiments thereof, it will be appreciated that those skilled in the art, upon attaining an understanding of the foregoing will readily conceive of alterations to, variations of and equivalents to these embodiments. Accordingly, the scope of the present invention should be assessed as that of the appended claims.

## Claims

1. A method of printing a moving substrate, comprising:
supplying a moving substrate (101) to a first converting operation (37);
contact printing at least one first graphic (80) on the moving substrate (101);
supplying the moving substrate (101) with the first graphic (80) to a second converting operation (39);
and non-contact printing at least one second graphic (82) on the moving substrate (101);
wherein the second converting operation (39) produces disposable absorbent garments, and the moving substrate forms an outer cover (20) of the garments, the outer cover (20) defining a width; and
wherein the first graphic (80) spans at least 60% of the width of the outer cover (20) and is visible to the naked eye, the second graphic (82) being positioned within the center third of the width of the outer cover (20) and being visible to the naked eye.

2. The method of claim 1, wherein the contact printing utilizes a gravure printer, flexographic printer, offset printer, or screen printer, the non-contact printing utilizes a wax jet printer, ink jet printer, laser jet printer, or bubble jet printer.

3. The method of claim 1 or claim 2, wherein the first graphic (80) and second graphic (82) jointly form a story line.

4. The method of any preceding claim, wherein the moving substrate (101) is traveling at least 0.508 m/sec (100 feet per minute) during the non-contact printing.

5. The method of any preceding claim, further comprising: non-contact printing at least one third graphic (84) on the moving substrate (101), wherein at least a portion of the third graphic (84) at least partially overprints the first graphic (80).

6. The method of any preceding claim, wherein the second graphic (82) at least partially overprints the first graphic (80).

7. The method of any preceding claim, wherein the first graphic (80) and the second graphic (82) jointly form a master graphic.

8. The method of any preceding claim, wherein the substrate (101) is a laminate comprising a film layer and a nonwoven layer and the first graphic (80) is printed on the film layer and the second graphic (82) is printed on the nonwoven layer.

9. The method of any of claims 1 to 7, wherein the substrate (101) is a laminate comprising a film layer and a nonwoven layer and the first graphic (80) is printed on the nonwoven layer and the second graphic (82) is printed on the nonwoven layer.

10. The method of any of claims 1 to 7, wherein the substrate (101) is a laminate comprising a film layer and a nonwoven layer and the first graphic (80) is printed on the film layer and the second graphic (82) is printed on the film layer.

11. The method of any preceding claim, wherein the absorbent garment has a front waist region (12), a back waist region (14), and a crotch region (16) connecting the front waist region (12) and the back waist region (14).

12. The method of claim 11, wherein the second graphic (82) is positioned within the front waist region (12).

13. The method of claim 11, wherein the second graphic (82) is positioned within the back waist region (14).

14. The method of claim 11, further comprising two or more second graphics (82), at least one second graphic (82) positioned within the front waist region (12) and at least one second graphic (82) positioned within the back waist region (14).

15. The method of any preceding claim, wherein the garment is a diaper, training pant, or adult incontinence garment.

16. The method of any of claims 1 to 15, wherein the method comprises:
laminating a first substrate comprising film to a second substrate comprising a nonwoven to form said outer cover (20), the outer cover (20) defining a film side and a nonwoven side opposite the film side;
supplying the outer cover (20) to said first converting operation and contact printing said at least one first graphic (80) on the nonwoven side,
supplying the outer cover (20) to said second converting operation, the second converting operation combining the outer cover (20) with an absorbent assembly to form the absorbent garment;
and non-contact printing said at least one second graphic (82) on the nonwoven side in the second converting operation.

## Patentansprüche

1. Ein Verfahren zum Bedrucken eines sich bewegenden Substrates, umfassend:
Zuführen eines sich bewegenden Substrates (101) zu einem ersten Verarbeitungsbetrieb (37);
Kontaktdrucken zumindest einer ersten Graphik (80) auf das sich bewegende Substrat (101);
Zuführen des sich bewegenden Substrates (101) mit der ersten Graphik (80) zu einem zweiten Verarbeitungsbetrieb (39);
und berührungsloses Drucken zumindest einer zweiten Graphik (82) auf das sich bewegende Substrat (101);
wobei der zweite Verarbeitungsbetrieb (39) ein absorbierendes Einwegkleidungsstück herstellt, und das sich bewegende Substrat eine äußere Abdeckung (20) des Kleidungsstückes bildet, wobei die äußere Abdeckung (20) eine Breite definiert; und
wobei sich die erste Graphik (80) über zumindest 60% der Breite der äußeren Abdeckung (20) erstreckt und mit bloßem Auge sichtbar ist, wobei die zweite Graphik (82) innerhalb des mittleren Drittels der Breite der äußeren Abdeckung (20) positioniert ist und mit bloßem Auge sichtbar ist.

2. Das Verfahren nach Anspruch 1, wobei das Kontaktdrucken einen Tiefdrucker, einen Flexodrucker, einen Offsetdrucker, oder einen Siebdrucker verwendet, und wobei das kontaktlose Drucken einen Wachsstrahldrucker, einen Tintenstrahldrucker, einen Laserdrucker, oder einen Bubble-Jet Drucker verwendet.

3. Das Verfahren nach einem der Ansprüche 1 oder 2, wobei die erste Graphik (80) und die zweite Graphik (82) zusammen eine Handlung bilden.

4. Das Verfahren nach einem der vorangehenden Ansprüche, wobei das sich bewegende Substrat (101) sich mit zumindest 0,508 m/sec (100 Fuß pro Minute) während des berührungslosen Druckens bewegt.

5. Das Verfahren nach einem der vorangehenden Ansprüche, weiterhin umfassend: berührungsloses Drucken zumindest einer dritten Graphik (84) auf das sich bewegende Substrat (101), wobei zumindest ein Teil der dritten Graphik (84) zumindest teilweise die erste Graphik (80) überdruckt.

6. Das Verfahren nach einem der vorangehenden Ansprüche, wobei die zweite Graphik (82) zumindest teilweise die erste Graphik (80) überdruckt.

7. Das Verfahren nach einem der vorangehenden Ansprüche, wobei die erste Graphik (80) und die zweite Graphik (82) zusammen ein Hauptgraphik bilden.

8. Das Verfahren nach einem der vorangehenden Ansprüche, wobei das Substrate (101) ein Laminat ist, das eine Filmschicht und eine Vliesschicht umfasst, und wobei die erste Graphik (80) auf der Filmschicht gedruckt ist und die zweite Graphik (82) auf der Vliesschicht gedruckt ist.

9. Das Verfahren nach einem der Ansprüche 1 bis 7, wobei das Substrat (101) ein Laminat ist, das eine Filmschicht und eine Vliesschicht umfasst und wobei die erste Graphik (80) auf der Vliesschicht gedruckt ist und die zweite Graphik (82) auf der Vliesschicht gedruckt ist.

10. Das Verfahren nach einem der Ansprüche 1 bis 7, wobei das Substrat (101) ein Laminat ist, das eine Filmschicht und eine Vliesschicht umfasst, und wobei die erste Graphik (80) auf der Filmschicht gedruckt ist und die zweite Graphik (82) auf der Filmschicht gedruckt ist.

11. Das Verfahren nach einem der vorangehenden Ansprüche, wobei das absorbierende Kleidungsstück einen vorderen Taillenbereich (12), einen hinteren Taillenbereich (14) und einen Schrittbereich (16) aufweist, der den vorderen Taillenbereich (12) und den hinteren Taillenbereich (14) verbindet.

12. Das Verfahren nach Anspruch 11, wobei die zweite Graphik (82) innerhalb des vorderen Taillenbereichs (12) positioniert ist.

13. Das Verfahren nach Anspruch 11, wobei die zweite Graphik (82) innerhalb des hinteren Taillenbereichs (14) positioniert ist.

14. Das Verfahren nach Anspruch 11, weiterhin umfassend zwei oder mehr zweite Graphiken (82), wobei zumindest eine zweite Graphik (82) innerhalb des vorderen Taillenbereichs (12) positioniert ist und zumindest eine zweite Graphik (82) innerhalb des hinteren Taillenbereichs (14) positioniert ist.

15. Das Verfahren nach einem der vorangehenden Ansprüche, wobei das Kleidungsstück eine Windel, ein Trainingshöschen, oder ein Inkontinenzkleidungsstück für Erwachsene ist.

16. Das Verfahren nach einem der Ansprüche 1 bis 15, wobei das Verfahren umfasst:
Schichten eines ersten Substrates umfassend einen Film auf ein zweites Substrat umfassend ein Vlies, um die äußere Abdeckung (20) zu bilden, wobei die äußere Abdeckung (20) eine Filmseite und eine Vliesseite gegenüber der Filmseite definiert;
Zuführen der äußeren Abdeckung (20) zum ersten Verarbeitungsbetrieb und Kontaktdrucken der zumindest einen ersten Graphik (80) auf die Vliesseite;
Zuführen der äußeren Abdeckung (20) zum zweiten Verarbeitungsbetrieb, wobei der zweite Verarbeitungsbetrieb die äußere Abdeckung (20) mit einer absorbierenden Anordnung kombiniert, um das absorbierende Kleidungsstück zu bilden;
und berührungsloses Drucken der zumindest einen zweiten Graphik (82) auf die Vliesseite im zweiten Verarbeitungsbetrieb.

## Revendications

1. Procédé d'impression d'un substrat en mouvement, comprenant :
la fourniture d'un substrat en mouvement (101) à une première opération de transformation (37) ;
l'impression par contact d'au moins un premier graphisme (80) sur le substrat en mouvement (101) ;
la fourniture du substrat en mouvement (101) pourvu du premier graphisme (80) à une seconde opération de transformation (39) ; et
l'impression sans contact d'au moins un second graphisme (82) sur le substrat en mouvement (101) ;
procédé dans lequel la seconde opération de transformation (39) produit des vêtements absorbants jetables, et le substrat en mouvement forme une feuille extérieure de couverture (20) des vêtements, la feuille extérieure de couverture (20) définissant une largeur ; et
dans lequel le premier graphisme (80) affecte au moins 60 % de la largeur de la feuille extérieure de couverture (20) et est visible à l'oeil nu, le second graphisme (82) étant positionné au sein du tiers central de la largeur de la feuille extérieure de couverture (20) et étant visible à l'oeil nu.

2. Procédé selon la revendication 1, dans lequel l'impression par contact utilise une presse à rotogravure, une imprimante flexographique, une imprimante offset, une imprimante sérigraphique, et l'impression sans contact utilise une imprimante à sublimation, une imprimante à jet d'encre, une imprimante laser ou une imprimante à bulles d'encre.

3. Procédé selon la revendication 1 ou la revendication 2, dans lequel le premier graphisme (80) et le second graphisme (82) forment ensemble le fil d'une histoire.

4. Procédé selon l'une quelconque des revendications précédentes, dans lequel le substrat en mouvement (101) se déplace à au moins 0,508 m/s (100 pieds/min) au cours de l'impression sans contact.

5. Procédé selon l'une quelconque des revendications précédentes, comprenant, en outre : l'impression sans contact d'au moins un troisième graphisme (84) sur le substrat en mouvement (101), procédé dans lequel au moins une portion du troisième graphisme (84) surimpressionne au moins partiellement le premier graphisme (80).

6. Procédé selon l'une quelconque des revendications précédentes, dans lequel le second graphisme (82) surimpressionne au moins partiellement le premier graphisme (80).

7. Procédé selon l'une quelconque des revendications précédentes, dans lequel le premier graphisme (80) et le second graphisme (82) forment ensemble un graphisme principal.

8. Procédé selon l'une quelconque des revendications précédentes, dans lequel le substrat (101) est un stratifié comprenant une couche de film et une couche de non-tissé, le premier graphisme (80) étant imprimé sur la couche de film et le second graphisme (82) étant imprimé sur la couche de non-tissé.

9. Procédé selon l'une quelconque des revendications 1 à 7, dans lequel le substrat (101) est un stratifié comprenant une couche de film et une couche de non-tissé, le premier graphisme (80) étant imprimé sur la couche de non-tissé et le second graphisme (82) étant imprimé sur la couche de non-tissé.

10. Procédé selon l'une quelconque des revendications 1 à 7, dans lequel le substrat (101) est un stratifié comprenant une couche de film et une couche de non-tissé, le premier graphisme (80) étant imprimé sur la couche de film et le second graphisme (82) étant imprimé sur la couche de film.

11. Procédé selon l'une quelconque des revendications précédentes, dans lequel le vêtement absorbant a une région de ceinture avant (12), une région de ceinture arrière (14) et une région d'entrejambe (16), connectant la région de ceinture avant (12) et la région de ceinture arrière (14).

12. Procédé selon la revendication 11, dans lequel le second graphisme (82) est positionné au sein de la région de ceinture avant (12).

13. Procédé selon la revendication 11, dans lequel le second graphisme (82) est positionné au sein de la région de ceinture arrière (14).

14. Procédé selon la revendication 11, comprenant, en outre, deux seconds graphismes (82), ou davantage, au moins un second graphisme (82) étant positionné au sein de la région de ceinture avant (12) et au moins un second graphisme (82) étant positionné au sein de la région de ceinture arrière (14).

15. Procédé selon l'une quelconque des revendications précédentes, dans lequel le vêtement est une couche-culotte, une culotte d'apprentissage de la propreté ou un vêtement pour l'incontinence adulte.

16. Procédé selon l'une quelconque des revendications 1 à 15, comprenant :
la stratification d'un premier substrat comprenant un film et un second substrat comprenant un non-tissé pour former ladite feuille extérieure de couverture (20), la feuille extérieure de couverture (20) définissant une face film et une face non-tissé opposée à la face film ;
la fourniture de la feuille extérieure de couverture (20) à ladite première opération de transformation et l'impression par contact dudit au moins un premier graphisme (80) sur la face non-tissée ;
la fourniture de la feuille extérieure de couverture (20) à ladite seconde opération de transformation, la seconde opération de transformation combinant la feuille extérieure de couverture (20) à un ensemble absorbant pour former le vêtement absorbant ; et
l'impression sans contact dudit au moins un second graphisme (82) sur la face non-tissée, dans la seconde opération de transformation.
